# EUROPEAN PATENT APPLICATION

(11) **EP 4 052 636 A1**
(43) Date of publication of application: **07.09.2022**
(21) Application number: 21159875.0
(22) Date of filing: 01.03.2021
(51) Int. Cl.: A61B 5/00, A61B 5/055, A61B 5/16

(54) **A SYSTEM FOR TREATING VISUAL NEGLECT**

(71) Applicant: Koninklijke Philips N.V., 5656 AG Eindhoven (NL)
(72) Inventor: VAN EE, Raymond, 5656 AE Eindhoven (NL)
(74) Representative: Philips Intellectual Property & Standards

(57) **Abstract**

A system for treating visual neglect comprises a virtual reality headset and an input for receiving functional MRI, fMRI, scanner images. During an initial analysis phase, visual images are generated for presentation to the user via the virtual reality headset at different angular and depth positions relative to the user. The fMRI images are processed to monitor brain activity relating to visual perception, eye movement generation and visual awareness of an object in response to the generated visual images. In this way, a 3D map is derived of the visual awareness of the user, for use in treating the visual neglect.

## Description

### FIELD OF THE INVENTION

The invention relates to a system for treating visual neglect.

### BACKGROUND OF THE INVENTION

Visual neglect involves a failure to perceive, to respond to, or to orientate the eyes to stimuli, typically in one half-field (left or right) of visual space. Visual neglect is usually caused by stroke and is accompanied by functional disability.

It is well known that depression can be a mental result of the physiological/neuronal impairment caused by visual neglect. Visual neglect involves about 400,000 new patients per year in the USA alone. In about 30-50% of visual neglect patients, major depression occurs as a result of the visual functional disability.

There is a widely acknowledged need to reduce the care burden of this depression.

The currently prevailing treatment of visual neglect (and the associated depression) uses prism training, involving an optical deviation to the left or right, induced by a fixed prism.

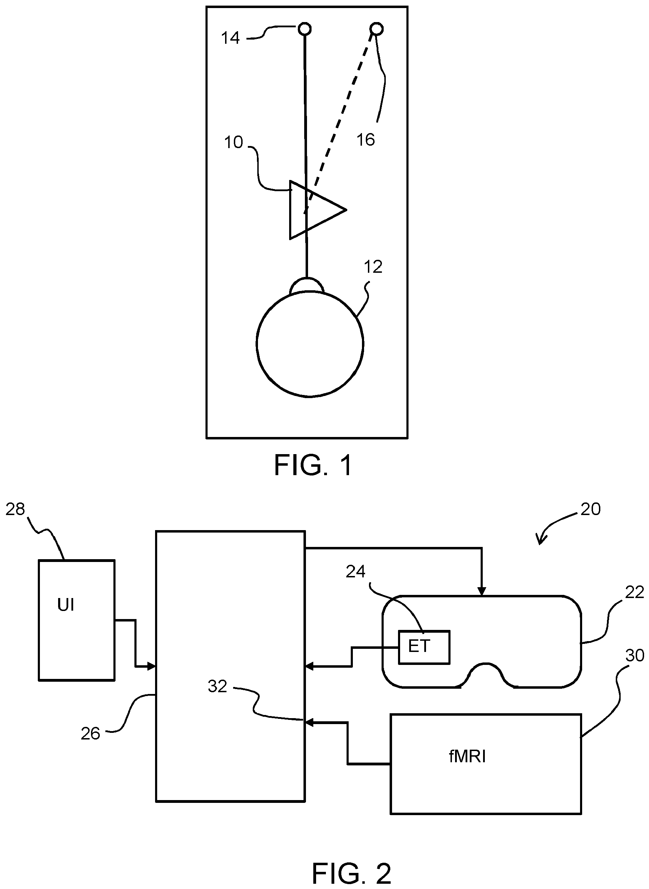

Figure 1 shows the use of a prism 10 in the line of sight of a patient 12 to modify the brain's perception of straight ahead.

During a training period, the brain adapts to a shift whereby an object 14 directly in front appears to be positioned to the right (at position 16). Initially, the patient experiences the central object as being positioned to the right. However, the brain adapts to this mismatch over time, so that the visual world is shifted to the left after the training period, meaning that the observer is then better able to perceive targets in the left visual hemisphere (which was previously neglected).

Training using such an optical deviation can rehabilitate visual neglect, leading to decreased severity of depression. In this treatment, visual neglect patients sit at a table (or sit up in bed) and are trained to make repeated visually guided pointing movements with their hand while wearing the wedge prisms shown in Figure 1, that laterally shift the external world.

On the brain's neuronal level, shifted visual field training modifies the way that visual information is transformed from retina-based coordinates to the arm's motor coordinates. Visual neglect patients wearing prisms that displace visual information rightward must make pointing movements to the left of where an object 'appears', to point at its actual location. In patients with neglect, this increase in e.g. leftward movement persists after the prism adaptation period, and can generalize to daily life tasks.

Currently a medical therapist must completely rely on the participant's responses as to whether training results in decreasing visual neglect. The conventional prism lens must be mounted in a spectacle frame, meaning that prism lens power is fixed and that during training the lens power stays fixed. It is not practical to adaptively change the lens power to increasing strength during the course of training.

It is known from rehabilitation practice that it is of importance for optimal training that the eyes' motor-skill system, in addition to the arm's motor-skill system, is being trained to recalibrate perceived straight ahead. However, there is a problem that a therapist is unable to accurately follow eye movements, so such a measurement would be impractical in the traditional (human guided) training. To a certain extent, the human trainer uses the arm motion as a proxy measure of the eye motion of the patient but this method is particularly imprecise in the neglected visual field.

It is known to use a virtual reality headset to simulate a prism offset, for example as disclosed in M. Wilf et. al., "Combined virtual reality and haptic robotics induce space and movement invariant sensorimotor adaptation", Neuropsychologia 150 (2021) 107692. This is more convenient than a prism system.

There remains a need for an improved system for treating visual neglect.

### SUMMARY OF THE INVENTION

The invention is defined by the claims.

According to examples in accordance with an aspect of the invention, there is provided a system for treating visual neglect, comprising:
a virtual reality headset;
an input for receiving functional MRI, fMRI, scanner images; and
a processor, wherein the processor is adapted to:
   in an initial analysis phase, generate visual images for presentation to the user via the virtual reality headset at different angular and depth positions relative to the user;
   process the fMRI images in real time thereby to monitor brain activity relating to visual perception, eye movement generation and visual awareness of an object in response to the generated visual images; and
   derive a 3D map of the visual awareness of the user, for use in treating the visual neglect.

This system makes use of a virtual reality headset to enable visual stimuli to be provided at different locations in a 3D volume forming the field of view of the user, including angular position (typically left-right but also optionally including up-down) as well as depth. There is now evidence that neglect can occur in only one region of space. This region could be peri-personal space (within arms' length) or extra-personal space (exceeding arms' length). A number of patients show neglect in both regions (peri-personal and extra-personal space). In a recent study, it has been found that 8% to 22% of patients showed neglect at one distance (i.e., peri-personal or extra-personal neglect), whereas 11% to 14% of patients showed neglect for both distances. When diagnosing neglect, it is relevant to distinguish the type of region-specific neglect and, where needed, to adjust the rehabilitation program accordingly. This is enabled in a simple way by the system of the invention.

The use of a virtual reality headset also addresses another existing shortcoming in current practice. Most studies use a visual barrier (a shelf, cardboard or cloth) to prevent the patients from continuously monitoring their own arm movements while they undergo training. This is impractical and it is hard to make the method adhere to set medical standards.

It is noted that by "virtual reality headset" is meant a display system worn by the user which blocks a view of the real world and displays a representation of a 3D virtual world. It could be glasses, or goggles or a helmet.

The system uses use functional brain processing data from the fMRI imaging (that is, functional brain areas or functional neuronal networks related to visual neglect) to assess the visual awareness. This may be used to derive a treatment program which optimizes the sensory stimulation for the treatment of visual neglect (so providing patient-tailored therapy), and also to monitor the effectiveness of previous training. The use of real time magnetic resonance imaging brain monitoring means a therapist is able to know whether the brain is able to pick up visual signals from the neglected past of visual space more accurately than form patient feedback alone.

The virtual reality headset provides an alternative way to implement a prism lens set up. It avoids the need to use a physical lens that must be mounted in a spectacle frame and it means that different lens powers can be emulated, including changes in lens power during training.

The processor may be adapted to derive a patient-tailored prism therapy from the initial analysis phase. The prism therapy is however implemented by the virtual reality headset rather than by physical prisms. The patient-tailored prism therapy for example comprises a virtual lens power setting to be simulated by the VR headset during the prism therapy. The patient-tailored prism therapy may comprise a virtual lens power which evolves over the duration of the prism therapy. This provides a dynamic therapy, which can achieve more rapid positive therapy outcomes.

The system preferably further comprises an eye movement tracking system integrated into the virtual reality headset.

Existing prism adaptation methods capitalize solely on a dissociation between perceived straight ahead and the arm's motor-skills system. The use of eye tracking means that, in addition to a dissociation between perceived straight ahead and the arm's motor system, the system can capitalize on a dissociation between perceived straight ahead and the eyes' motor system.

Indeed, it is known from rehabilitation practice that it is of interest for optimal training that the eyes' motor-skill system, in addition to the arm's motor-skill system, is being trained to recalibrate perceived straight ahead. The technical ability to measure the posture and the movements of the eye is therefore of interest. In a conventional prism-adaptation set-up eye movements are not measured. A therapist is unable to accurately follow eye movements so such a measurement is impractical in the traditional (human guided) training. Instead, the arm motion is used as a proxy measure of the eye motion of the patient but this method is particularly imprecise in the neglected visual field.

The system enables eye tracking in the MR imaging bore to optimize the patient-tailored prism therapy which can then result in a more effective treatment of depression.

The processor may be adapted to process the eye movement tracking information to confirm the direction and depth of the gaze of the user. Thus, the eye tracking can determine both the angle and depth of the focus of the patient.

The use of eye tracking may enable dissociation to be generated between a visible hand location (as presented to the user as a virtual image) and the motor coordinates of the eye, or dissociation between a visible object location (again presented as a virtual image) and the motor coordinates of the eye. Dissociation may also be generated between a visible hand location and a visible object location.

For example, in a therapy routine, image generation may be controlled in real time in response to the eye tracking information, for example to move an image of an object (or face or hand image) such that it remains ahead of the gaze of the patient. This may provide a more challenging (and hence more stimulating) experience, and consequently a more effective treatment for the patient. This provides additional flexibility compared to known systems which generate only a dissociation between the visible hand location and the arm motor coordinates.

The processor is for example adapted to generate visual images which move from a region for which the user is visually aware to a region for which the user is not visually aware. The fMRI analysis can then track when the user ceases to perceive the presented image.

The processor may be adapted, in the initial analysis phase, to display a face at a selected angle and depth, the face to be recognized by the user, and determine if the face has been seen and recognized by the monitored brain activity.

The use of a recognizable face stimulates the prefrontal cortex and this stimulates a stronger brain response to the visual images. The image of a face may also be moved as explained above from the visible field to the neglected field.

The system may further comprise an input for receiving an indication from the user that the face has been seen and recognized. This provides additional feedback to make the processing more robust.

The processor may be adapted to process the fMRI images to monitor brain activity monitored in one or more of the visual cortex, the FusiForm face area, the prefrontal cortex and the frontal cortex.

The system may further comprise the fMRI scanner.

The processor is for example further adapted, in a treatment phase, to:
generate visual images for presentation to the user via the virtual reality headset which introduce a shift of the real visual world that would be present in the field of view of the subject without wearing the virtual reality headset, wherein the shift is in both angular and depth directions.

This provides a virtual reality implementation of prism therapy.

The invention also provides a computer-implemented method for collecting data for use in treating visual neglect, comprising:
generating visual images for presentation to the user via a virtual reality headset at different angular and depth positions relative to the user;
processing fMRI images thereby to monitor brain activity relating to visual perception, eye movement generation and visual awareness of an object in response to the generated visual images;
deriving a 3D map of the visual awareness of the user, for use in treating the visual neglect.

The method may further comprise deriving a patient-tailored prism therapy from the initial analysis phase.

The invention also provides a computer program comprising computer program code means which is adapted, when said program is run on a computer, to implement the method defined above.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows the use of a prism in the line of sight of a patient to modify the brain's perception of straight ahead;
Figure 2 shows a system for treating visual neglect;
Figure 3 shows the result of an experiment to show the applicability of using virtual reality to simulate a prism shift; and
Figure 4 shows a computer-implemented method for collecting data for use in treating visual neglect.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for treating visual neglect, comprising a virtual reality headset and an input for receiving functional MRI, fMRI, scanner images. During an initial analysis phase, visual images are generated for presentation to the user via the virtual reality headset at different angular and depth positions relative to the user. The fMRI images are processed to monitor brain activity relating to visual perception, eye movement generation and visual awareness of an object in response to the generated visual images. In this way, a 3D map is derived of the visual awareness of the user, for use in treating the visual neglect.

Figure 2 shows a system 20 for treating visual neglect. The system comprises a virtual reality headset 22 which includes an eye tracking system 24. The system further comprises a processor 26 and a user interface 28 for receiving control commands from the user and generating an output for the user.

The system is for use in conjunction with a functional MRI, fMRI, scanner 30, and the processor 26 has an input 32 for receiving fMRI images.

The processor 26 is used both for an initial analysis phase and for a treatment phase. During the initial analysis phase, the condition of a patient is assessed. This involves generating visual images for presentation to the user via the virtual reality headset 22 at different angular and depth positions relative to the user. The aim is to identify the regions 3D in space for which the patient suffers from visual neglect.

In order to make this identification, the fMRI images are processed in real time thereby to monitor brain activity relating to visual perception, eye movement generation and visual awareness of an object in response to the generated visual images.

For the analysis phase, the patient is positioned within the MR scanner 30.

One example of how to implement the analysis phase is to present a picture of a familiar face within the visual field of the patient and to measure, using real-time brain activity, whether the brain is able to partially or fully process the visual stimulus.

An important brain area of which the activity is being monitored is the visual cortex in order to test whether the presented picture is detected, first by the eyes, and subsequently by the brain. In addition, the FusiForm-Face area of the brain may also be monitored in order to check whether the face is being processed by face-processing brain areas.

Brain activity in the visual cortex and in the FusiForm-Face area does not however provide proof that the patient is both aware of the face picture and that the patient can orient either the eyes or attention to parts of the face. Monitoring of the prefrontal cortex for attention related brain areas, and eye movement related brain areas, may be performed to ensure that the visual object resulted in awareness of the face picture by the patient.

The patient may also be instructed to provide a user input via the user interface 28, for example by pressing a button when the presented visual object is perceived (meaning that the patient is aware of the presented visual object) or when the object is no longer perceived. The measurement of brain activity may be repeated for locations of the face picture throughout the external 3D visual field in front of the patient's head. This then results in a 3D map consisting of locations where the patient is not aware of the face-picture.

This procedure can be automated. For example, an automated system can be used to map a 2D frontal plane where targets are either visible or neglected. An artificial intelligence agent can be used to adaptively change the projected distance of the targets in the visual field to provide a systematic diagnosis for distance-dependent visual neglect. The analysis may simulate distances both within arms' length and outside arms' length.

By way of example, for patients with visual neglect in the left visual hemifield, vision in the right hemifield is usually unaffected. For such patients, a target maybe presented that moves from the far right visual hemifield (the good field) to the left into the left visual hemifield (the neglected field). The start is at the right side for a patient with left visual neglect because starting at the left side would mean that the target is invisible to begin with. By monitoring each of the visual-processing related brain areas it is possible to objectively monitor whether presentation of the face-picture in a particular location in the 3D visual field leads to awareness. Feedback from the patient may also be used, which in this example will be the moment at which the target becomes invisible.

A 3D map is thus derived of the visual awareness of the user in the analysis phase, for use in treating the visual neglect in the subsequent treatment phase. The virtual reality headset is able to generate visual stimuli to at different locations in a 3D field of view volume, i.e. including angular position (typically left-right but also optionally including up-down) as well as depth.

It is particularly interesting to map those parts of the 3D visual field where the real-time fMRI indicates to the therapist that the face-picture is fully processed, while the patient is not yet aware of the face-picture. In this part of the visual field, the likelihood for a successful therapy is highest. For this part of the visual field, the therapist is guided in the treatment phase to repeat presentation of the visual object until sufficient attentional resources (and corresponding neuronal synaptic connections) are recruited to learn to perceive the visual object and to overcome visual neglect for that particular part of 3D visual space in front of the head of the patient.

The real time processing of fMRI images means functional brain processing data (that is, functional brain areas or functional neuronal networks related to visual neglect) are used to assess the visual awareness.

The subsequent treatment phase involves deriving a treatment program which optimizes the sensory stimulation for the treatment of the identified visual neglect. The therapy is thus patient-tailored. The analysis phase may be repeated to monitor the effectiveness of previous training.

The treatment phase may be performed entirely by the virtual reality headset, i.e. without the fMRI feedback, so that the fMRI feedback is only used for the mapping. The treatment phase may for example be performed at home. However, the fMRI feedback may also be used during the treatment phase to optimize the feedback of the patient response obtained during treatment.

The virtual reality headset is for example used to create a lateral shift between the visual direction of a perceived object and an object that could be pointed at using the real hand. It has been established by experiment that a virtual reality headset is effective in evoking a shift of the visual world relative to the motor space.

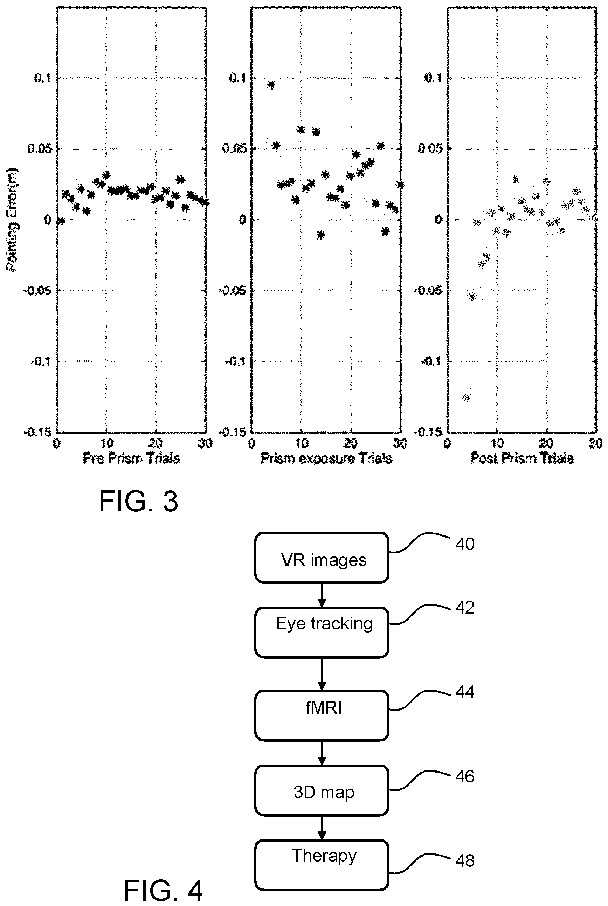

Figure 3 shows the result of the experiment to verify the effectiveness of virtual reality for simulating a prism shift. Each image plots the pointing error for a series of successive trials, i.e. over time. The left image shows the hand pointing error for a session using a virtual reality headset but without any lateral shift of the projected visual world. The middle image shows pointing error data when implementing a lateral shift of the projected visual world (mimicking a prism shift). At the first pointing event, the individual points his hand 10 cm to the right of the object. Because the hand is visible (as a virtual image generated by the virtual reality headset) the individual adapts to the new perceived shift of the object after a few pointing trials.

The right image shows pointing data after the lateral shift is suddenly nulled again. This time the individual, being adapted to the shift, points about 10 cm to the left. After a few trials the individual adapts again to the zero lateral shift.

The use of eye tracking with the virtual reality headset addresses the issue that a trainer is not able to follow eye movement with any accuracy. The eye movement measurement may be made using already commercially available virtual reality headsets. The direction and depth of focus can be tracked in real time. The 3D images presented to the user may also be positioned at any desired angular position and depth position within the field of view of the user, so that adaptive and personalized modification of the projected distance of the targets in the visual field as well as lateral shift can be achieved. Thus, the analysis enables evaluation of distance dependent as well as lateral visual neglect.

The virtual reality headset may be used to project an artificial hand, or artificial hands to the user, as well as other objects. Training objects can be projected at the distance and region that was identified during the analysis stage. For example, a visual object as well as images of (artificial) hands may be shifted over a visual angle so that a prism shift is mimicked.

The visual neglect training essentially involves selecting a simulated lens power for the training. At first, this selection can be based upon a database that contains a recommended lens power fitting personal characteristics of the patient (such as age, or period of time after stroke, etc.). The visual neglect training may also be automated.

The training routine may involve applying a virtual lens power which evolves over the duration of the prism therapy. This may be automatic or the adjustments may be made by a therapist to personalize and adapt to the patient's capacities. The personalized virtual lens power may adapt during training in order to make the visual deviation (the virtual prism lens power) increasingly challenging. This can be done in a straightforward way when using the virtual reality headset.

The tracking of eye movements may be used to make the therapy more effective. The eye movements may for example be tracked while the eyes attempt to follow a target that moves (e.g. from right to left in the visual field) during the therapy. The target position may be shifted in real time relative to the measured eye posture so that the eye will not be able to exactly follow the target. At the same time, the mapping of brain areas (prefrontal cortex) related to eye movements to the targets may be maintained using the functional MR imaging in cases where the therapy is conducted in combination with the fMRI imaging.

For example, in a first therapy session within the scanner, the patient may be asked to follow a target (using eye movements to keep fixation on the target) in the visual field of a whole-field virtual reality set-up in which the target moves from the far right visual hemifield to the left into the left visual hemifield. The stimulation with this moving target is repeated a predetermined number of times (e.g. ten repetitions).

The eye tracking may first take place without any visual shift. The patient may for example be able to keep an awareness of the target, and to follow the target through eye movement, until it moves X degrees (from the midline to the left) into the neglected field.

In a subsequent stimulation session, the visual shift manipulation may be performed in order to teach the patient's brain to make eye movements deeper into the neglected field. Thus, the aim is to increase the value of X by means of the training. To make this adaptation work, the patient's brain needs to be taught that the visual midline relative to the rest of the body is shifted just as in the traditional prism adaptation method. However, the eye tracking means the eye motor system is used. The target (object or face) is for example continuously projected Z degrees to the left of where the patient is pointing with the eyes. The patient is however asked to carefully attempt to fixate the eyes on the target and to follow the target into the left visual hemifield. This stimulation with the moving target is also repeated a predetermined number of times (e.g. ten repetitions).

The patient may then be able, over time, to keep awareness of the target, and to follow the target through eye movements, until the eyes move X+Z degrees (from the midline to the left) into the neglected field.

In a further stimulation session, the task is repeated without simulated visual shift. The patient will now be able to keep awareness of the target until it moves X+Z degrees into the neglected field.

The example above is based on eye tracking and fMRI monitoring to enable dissociation to be generated between a visible object location and the motor coordinates of the eye.

Another example is to enable dissociation to be generated between a visible hand location or arm posture (with the hand/arm presented to the user as a virtual image) and the motor coordinates of the eye. The patient may use hand movements to follow the target.

The therapy may then follow the same general approach as outlined above. In a first therapy session within the scanner, the patient is for example asked to follow a target using pointing hand movements in the visual field of a whole-field virtual reality set-up. The target may again move from the far right visual hemifield to the left into the left visual hemifield (or the other way around for neglect on the opposite side).

The stimulation may be repeated with the moving target a predetermined number of times (e.g. ten repetitions).

As in the example above, the stimulation is first performed without a visual shift. The patient may again be able to keep awareness of the target until it moves X degrees (from the midline) into the neglected field.

As in the example above, the visual shift manipulation is then performed in order to teach the patient's brain to make hand movements deeper into the neglected field (X must increase due to training). The visual shift manipulation in this case is implemented by projecting an artificial representation of the patient's hand in the whole field of the virtual reality environment. The projected hands are shifted relative to the real midline by Y degrees.

The same target is then presented as above without the visual field shift, again moving from the far right visual hemifield to the left into the left visual hemifield. The patient is asked to point the artificial hand (shifted in position relative to the real hand, meaning that the virtual hand is projected based on the position of the real hand) and to follow both the target and the hand by eye movements.

In this way the eye movement motor system is dissociated from the hand-arm-body motor system. This recalibration of the hand motor system is the key to teaching the patient's brain to make hand movements deeper into the neglected field than prior to the adaptation to the visual field shift. By repeating this target motion a predetermined number of times, the patient is trained to adapt to a shift in the projection of the hand relative to the visual field of Y degrees. Note that if the hand would not be visible in the virtual reality environment, there would be no incentive for the eye motor system to recalibrate its orientation relative to the body because a person is then not able to know where the eyes point relative to the head.

As in the example above, if the first task is repeated without the prism adaptation, the patient should then able to keep awareness of the target until it moves X+Y degrees into the neglected field.

The dissociation between the hand and eye motor system and between the object and eye motor system may be combined within an overall therapy. Furthermore, dissociation may be generated between the hand and an object as another therapy option.

The treatment therapy may include a reward system (e.g. by a piece of music or other notification) whereby the user is rewarded for successful treatment outcomes.

Figure 4 shows a computer-implemented method for collecting data for use in treating visual neglect. The method comprises:
in step 40, generating visual images for presentation to the user via a virtual reality headset at different angular and depth positions relative to the user;
in step 42, tracking eye movements to determine a point of focus in 3D space;
in step 44, processing fMRI images thereby to monitor brain activity relating to visual perception, eye movement generation and visual awareness of an object in response to the generated visual images;
in step 46, deriving a 3D map of the visual awareness of the user, for use in treating the visual neglect; and
in step 48, deriving a patient-tailored prism therapy from the initial analysis phase.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for treating visual neglect, comprising:
a virtual reality headset (22);
an input (32) for receiving functional MRI, fMRI, scanner images; and
a processor (26), wherein the processor is adapted to:
in an initial analysis phase, generate visual images for presentation to the user via the virtual reality headset at different angular and depth positions relative to the user;
process the fMRI images in real time thereby to monitor brain activity relating to visual perception, eye movement generation and visual awareness of an object in response to the generated visual images; and
derive a 3D map of the visual awareness of the user, for use in treating the visual neglect.

2. The system of claim 1, wherein the processor is adapted to derive a patient-tailored prism therapy from the initial analysis phase.

3. The system of claim 2, wherein the patient-tailored prism therapy comprises a virtual lens power setting to be simulated by the VR headset during the prism therapy.

4. The system of claim 3, wherein the patient-tailored prism therapy comprises a virtual lens power which evolves over the duration of the prism therapy.

5. The system of any one of claims 1 to 4, further comprising an eye movement tracking system (24) integrated into the virtual reality headset.

6. The system of claim 5, wherein the processor (26) is adapted to process the eye movement tracking information to confirm the direction and depth of the gaze of the user.

7. The system of any one of claims 1 to 6, wherein the processor (26) is adapted to generate visual images which move from a region for which the user is visually aware to a region for which the user is not visually aware.

8. The system of any one of claims 1 to 7, wherein the processor (26) is adapted, in the initial analysis phase, to display an image in the form of a face, the face to be recognized by the user, and determine if the face has been seen and recognized by the monitored brain activity.

9. The system of claim 8, further comprising an input (28) for receiving an indication from the user that the face has been seen and recognized.

10. The system of any one of claims 1 to 9, wherein the processor (26) is adapted to process the fMRI images to monitor brain activity monitored in one or more of the visual cortex, the FusiForm face area, the prefrontal cortex and the frontal cortex.

11. The system of any one of claims 1 to 10, further comprising the fMRI scanner (30).

12. The system of any one of claim 1 to 11, wherein the processor (26) is further adapted, in a treatment phase, to:
generate visual images for presentation to the user via the virtual reality headset which introduce a shift of the real visual world that would be present in the field of view of the subject without wearing the virtual reality headset, wherein the shift is in both angular and depth directions.

13. A computer-implemented method for collecting data for use in treating visual neglect, comprising:
generating visual images for presentation to the user via a virtual reality headset at different angular and depth positions relative to the user;
processing fMRI images thereby to monitor brain activity relating to visual perception, eye movement generation and visual awareness of an object in response to the generated visual images;
deriving a 3D map of the visual awareness of the user, for use in treating the visual neglect.

14. The method of claim 13, further comprising deriving a patient-tailored prism therapy from the initial analysis phase.

15. A computer program comprising computer program code means which is adapted, when said program is run on a computer, to implement the method of claim 13 or 14.
